Europäisches Patentamt

European Patent Office

Office européen des brevets

(19)

(11) Numéro de publication : **0 480 853 A1**

(12) **DEMANDE DE BREVET EUROPEEN**

(21) Numéro de dépôt : **91420355.9**

(22) Date de dépôt : **08.10.91**

(51) Int. Cl.⁵ : **G01N 3/18**

(30) Priorité : **09.10.90 FR 9012765**

(43) Date de publication de la demande :
**15.04.92 Bulletin 92/16**

(84) Etats contractants désignés :
**AT BE CH DE DK ES FR GB GR IT LI LU NL SE**

(71) Demandeur : **IMAGO**
**8 Parc Cunéo d'Ornano**
**F-20000 Ajaccio (FR)**

(72) Inventeur : **Buffard, Laurent**
**Mas Lei Roumanieu, Quartier de la Bourrasque**
**F-83270 Saint Cyr Sur Mer (FR)**
Inventeur : **Raymond, Michel**
**Le Majorque, Baie des Anges**
**F-13600 La Ciotat (FR)**
Inventeur : **Girones-Weynant, Renée-Paule**
**Villa Coste Gaye, Route de Lauris**
**F-84160 Cadenet (FR)**
Inventeur : **Weynant, Eric**
**Villa Coste Gaye, Route de Lauris**
**F-84160 Cadenet (FR)**

(74) Mandataire : **Maureau, Philippe et al**
**Cabinet GERMAIN & MAUREAU BP 3011**
**F-69392 Lyon Cédex 03 (FR)**

(54) **Machine pour le contrôle des caractéristiques de matériaux présentant une transformation de phase, réversible ou non.**

(57)    Cette machine comprend une enceinte (2) isolée thermiquement, à laquelle sont associés une unité de commande, un générateur de température (10, 32) un dispositif (14) d'homogénéisation de la température, un porte-échantillons (45) disposé à l'intérieur de l'enceinte, et des organes de mesure, des moyens étant prévus pour assurer le montage des différents éléments de commande ou de contrôle fixés sur l'enceinte, tout en permettant les dilatations thermiques de l'enceinte, sous l'effet des différences de température.

Application au contrôle d'éléments en alliages à mémoire de forme.

FIG.1

EP 0 480 853 A1

La présente invention a pour objet une machine pour le contrôle des caractéristiques de matériaux présentant une transformation de phase, réversible ou non.

Cette machine est destinée notamment au contrôle des éléments en alliages à mémoire de forme qui présentent une transformation martensitique, mais aussi d'autres matériaux, tels que des céramiques ou des polymères.

Les alliages à mémoire de forme se transforment d'une phase haute température à une phase basse température, lors du passage à une température déterminée. C'est de cette transformation que les alliages à mémoire de forme tirent leurs propriétés de mémoire de forme. De ces propriétés, il résulte que l'on peut donner à tout objet en alliage à mémoire de forme, deux formes : l'une à basse température, l'autre à haute température.

Cette transformation est également sensible aux contraintes mécaniques appliquées au matériau : plus la contrainte est élevée, plus la température de transformation augmente.

Il est donc intéressant de pouvoir mesurer un effort ou une déformation sur ces alliages, ou de vérifier la régularité des caractéristiques d'éléments en alliages à mémoire de forme. Ces mesures de déformation mécanique peuvent être avantageusement complétées par des mesures d'autres paramètres caractéristiques de la transformation matensitique, tels que la résistivité électrique et l'émission acoustique.

Il n'existe pas, actuellement, de machines convenant pour effectuer de telles mesures, ces machines apparaissant difficiles à réaliser pour donner des résultats fiables dans une large plage de températures de l'ordre de - 150°C à + 300°C, compte tenu des problèmes de dilatations, de contraintes thermiques, et de tenue en température des divers instruments de mesure.

Le but de l'invention est de fournir une machine susceptible de soumettre au moins un échantillon à une contrainte, ou à une déformation donnée, dans une large plage de températures, par exemple de - 150°C à + 300°C, et de mesurer notamment la force exercée sur les échantillons, la déformation des échantillons, la température des échantillons, ainsi qu'éventuellement d'autres paramètres, tels que résistivité électrique et émission acoustique.

A cet effet, la machine qu'elle concerne, comprend une enceinte isolée thermiquement, à laquelle sont associés une unité de commande, un générateur de température, un dispositif d'homogénéisation de la température, un porte-échantillons disposé à l'intérieur de l'enceinte et des organes de mesure, des moyens étant prévus pour assurer le montage des différents éléments de commande ou de contrôle fixés sur l'enceinte, tout en permettant les dilatations thermiques de l'enceinte, sous l'effet des différences de température.

Il est en effet important d'assurer une bonne tenue de ces éléments sans perturber les phénomènes de dilatations.

Selon une forme d'exécution de cette machine, l'enceinte isolée thermiquement comprend une cuve en acier de forme générale cylindrique comportant des fonds bombés vers l'extérieur, logée à l'intérieur d'une enveloppe métallique, avec interposition de matériau isolant rigide, l'un des fonds de la cuve étant équipé d'une part, de tubulures traversant avec jeu l'isolant de l'enveloppe et destinées respectivement à l'amenée de fluide cryogénique, au passage d'un arbre d'entraînement d'un ventilateur assurant la circulation de fluide gazeux à l'intérieur de l'enceinte et l'homogénéisation de la température, et à la régulation de pression à l'intérieur de l'enceinte, ces trois tubulures étant fixées également sur une pièce en forme d'étrier solidaire d'un anneau fixé sur la partie cylindrique de la cuve, à proximité de ce fond, et d'autre part, d'au moins une ouverture radiale pour le passage d'un porte-échantillons et de l'instrumentation de mesure, chaque ouverture étant délimitée par une manchette, les différentes manchettes étant solidaires d'un anneau qui entoure la cuve et qui est relié par des barres longitudinales à un deuxième anneau qui est monté de façon ajustée et avec possibilité de coulissement axial sur l'anneau portant l'étrier.

Cette structure avec des anneaux et barres de liaison permet d'assurer un montage solide des différents éléments avec un parfait positionnement, qui n'est pas perturbé par les phénomènes de dilatation, compte tenu des possibilités de coulissement des deux anneaux montés ajustés l'un dans l'autre.

Avantageusement, la cuve est d'axe horizontal, son enveloppe repose sur un support, et elle comporte deux ouvertures diamétrales pour le passage de l'instrumentation dont une partie est elle-même fixée sur le support.

La cuve ainsi que la structure qui lui est associée reposent sur le matériau isolant et n'ont qu'un point fixe par rapport à l'extérieur, constitué par le passage de l'instrumentation.

Selon une autre caractéristique de cette machine, l'enceinte thermique comporte une paroi cylindrique en tôle mince qui, s'étendant dans la partie cylindrique de la cuve, à l'intérieur de celle-ci, et délimitant une zone centrale et une zone annulaire, comporte des ouvertures pour le passage des échantillons et de l'intrumentation, le ventilateur étant disposé en regard de la zone centrale, le fond opposé à celui du côté duquel se trouve le ventilateur étant équipé d'un séparateur de flux favorisant le retour de l'air par la zone annulaire, une résistance de chauffage étant disposée dans la zone centrale, en aval du ventilateur, dans le sens d'écoulement du flux d'air.

Une sonde de température disposée en aval du ventilateur et de la résistance, est reliée au régulateur qui commande le générateur de température. S'il faut chauffer, le régulateur commande la mise en fonctionnement de la résistance. Si, au contraire, il faut refroidir, le régulateur commande l'injection de fluide cryogénique par une buse calibrée, à partir d'une électro-vanne proportionnelle.

La ventilation améliore :

– l'échange thermique entre la résistance de chauffage et l'air de l'enceinte,

– le mélange des fluides cryogéniques et gazeux et, notamment, l'évaporation du fluide cryogénique,

– l'échange thermique entre le fluide gazeux de l'enceinte et les échantillons.

Dans le fond de la cuve, opposé à celui traversé par l'arbre du ventilateur, est montée une soupape de sécurité limitant la pression interne à des valeurs de légère surpression de 1,1 à 1,2 bars.

Selon une autre caractéristique de l'invention, la manchette solidaire de la cuve et tournée vers le haut est prolongée par une partie tubulaire qui, traversant l'isolant et l'enveloppe extérieure, est équipée, à l'extérieur de cette dernière, d'une plate-forme sur laquelle est monté un dispositif élévateur destiné à déplacer l'instrumentation et les échantillons lors de leur mise en place dans la cuve et de leur retrait hors de celle-ci.

Avantageusement, le dispositif élévateur comprend un moto-réducteur entraînant une vis sans fin, d'axe parallèle à celui de l'instrumentation, sur laquelle est monté un écrou solidaire d'un chariot qui, guidé sur des rails parallèles à la vis, est bloqué en translation sur le bâti portant l'instrumentation, des capteurs inductifs étant prévus pour commander l'arrêt du moteur en fin de course haute et basse du chariot.

Le bâti portant l'instrumentation est équipé d'un étrier qui, en position de mesure, se trouve à l'intérieur de la cuve, et sert au montage de chaque élément à contrôler, par appui ou par fixation de l'extrémité de celui-ci opposée au côté duquel se trouve le dispositif élévateur, l'autre extrémité de chaque élément étant en appui contre ou fixé à l'extrémité d'une tige d'orientation générale radiale par rapport à l'enceinte, montée coulissante par rapport au bâti et dont l'autre extrémité, située du côté de l'élévateur, est équipée d'un dispositif permettant son déplacement axial et est associée à un capteur de force et à un capteur de déplacement.

Ce montage permet de réaliser les différents types d'essais suivants :

– Sur l'élément formant échantillon est appliquée une force constante, après quoi l'on mesure la variation de longueur en fonction d'une variation imposée de la température.

– A l'élément formant échantillon est imposée une variation de longueur, après quoi l'on mesure la force développée par l'échantillon lors d'une variation de température.

– La température de l'élément formant échantillon est fixée, après quoi l'on mesure la force nécessaire pour le déformer, à la traction ou à la compression.

Afin d'imposer une déformation ou une vitesse de déformation à un élément formant échantillon, l'extrémité de la tige située du côté opposé à l'élément à contrôler est fixée par l'intermédiaire d'un capteur de force à un dispositif vis-écrou, dont l'un des éléments est associé au support du capteur de force et dont l'autre élément est associé au bâti, afin de transformer une rotation imposée en translation.

Dans le cas où la machine est équipée pour contrôler deux éléments disposés parallèlement l'un à l'autre, les deux capteurs de force qui leur sont associés sont montés sur une platine unique déplaçable en translation de façon contrôlée, ce qui assure qu'une même déformation est appliquée aux deux échantillons.

La fiabilité des mesures est avantageusement augmentée en utilisant un fonctionnement différentiel. A cet effet, on dispose dans la machine deux éléments, l'un servant de référence et l'autre étant à caractériser.

Les capteurs de force utilisés sont par exemple du type jauges de contrainte.

Chaque tige dont une extrémité est associée à un élément formant échantillon est équipée, à proximité de son extrémité située à l'extérieur de l'enceinte, d'une lame qui lui est perpendiculaire et qui porte la partie mobile d'un capteur de déplacement.

Avantageusement, chaque capteur de déplacement est de type magnétique, et réagit au déplacement d'un noyau de fer doux, qui est solidaire de la tige dont le déplacement est à mesurer.

Afin d'éviter les déperditions thermiques hors de l'enceinte, et permettre un fonctionnement fiable de l'instrumentation malgré les grandes variations de température, la partie du bâti traversant l'enveloppe extérieure, le matériau isolant et la paroi de la cuve, est tubulaire et montée avec interposition de joints dans les éléments tubulaires fixes traversant ces éléments, cette partie tubulaire comportant elle-même un tube de guidage de chaque tige, dans lequel celle-ci est montée avec interposition de douilles à billes, un isolant thermique étant prévu entre la tige et les douilles, afin de protéger ces dernières des températures extrêmes pouvant régner à l'intérieur de l'enceinte.

Les instruments étant disposés à l'extérieur de l'enceinte ne sont pas soumis aux importantes variations de température.

Avantageusement, la machine est équipée, du côté de la cuve diamétralement opposé à celui comportant l'instrumentation de mesure des déformations, d'un dispositif de mesure d'émission acoustique, monté sur l'étrier portant la ou les éléments à contrôler, par l'intermédiaire d'un bâti comportant, dans sa zone de traversée de la paroi de la cuve, de l'isolant thermique et de l'enveloppe, une partie tubulaire montée avec interposition de joints dans les éléments tubulaires fixes traversant ces éléments.

Pour des raisons d'exactitude de la mesure et de fiabilité, le capteur d'émission acoustique est monté à l'extérieur de l'enceinte contenant l'élément à contrôler, les ondes ultra-sonores étant guidées de l'élément au capteur par un guide d'ondes en appui sous une force de tension réglable d'une part, sur l'élément et d'autre part, sur le capteur, des moyens étant prévus pour réaliser un montage découplant, d'une part, le guide d'ondes du bâti de la machine pour s'affranchir des vibrations parasites, et permettant, d'autre part, un réglage indépendant des pressions entre guide d'ondes et capteur et guide d'ondes et élément.

Le capteur d'émission acoustique est, par exemple, un capteur piézo-électrique, de type résonnant ou à large bande.

Les mesures d'émission acoustique permettent des mesures de grandeur des phénomènes dynamiques liés à la rupture, mais aussi aux déformations plastiques et à la transformation martensitique. Il est ainsi possible de déterminer, par cette mesure, la dynamique de la transformation martensitique. Ceci permet de contrôler l'histoire de l'alliage par rapport à la transformation martensitique et donc de prévoir le comportement futur de l'alliage.

Pour mesurer la résistivité, quatre contacts électriques sont réalisés sur l'élément par soudage par points. Cette mesure permet de suivre la transformation en déterminant, au fur et à mesure de celle-ci, le pourcentage transformé.

Afin de mesurer la température de l'élément à contrôler, de façon continue, un thermocouple est soudé à l'échantillon, par soudage par points.

De toute façon, l'invention sera bien comprise à l'aide de la description qui suit, en référence au dessin schématique annexé, représentant à titre d'exemple non limitatif, une forme d'exécution de cette machine.

Figure 1 en est une vue partielle en coupe longitudinale pour ce qui concerne l'enceinte thermique ;

Figure 2 est une vue en perspective éclatée et à échelle agrandie de la structure associée à l'enceinte thermique, destinée à porter les principaux éléments de l'instrumentation ;

Figure 3 est une vue en coupe par un plan perpendiculaire à l'axe de l'enceinte thermique, représentant le système de mise en contrainte, et de mesure mécanique des déformations ;

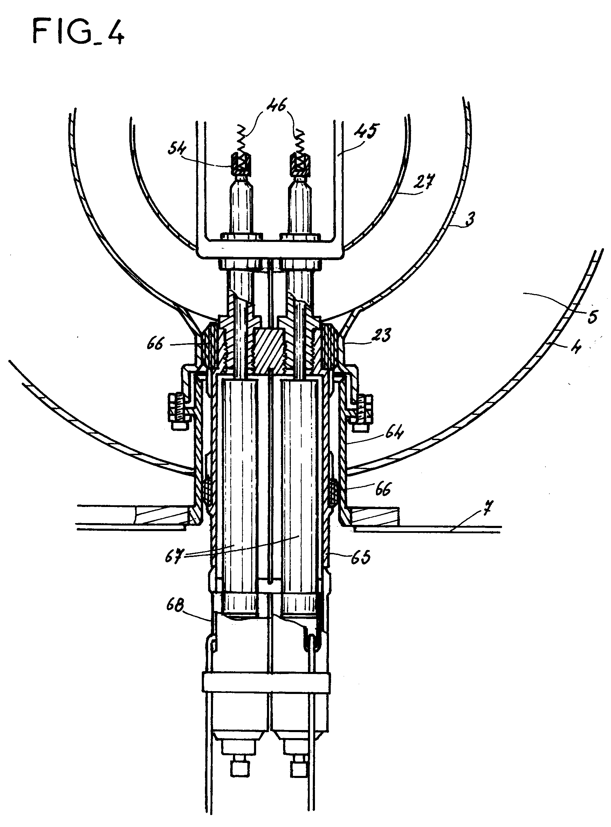

Figure 4 est une vue complémentaire de figure 3, en coupe par le même plan transversal à l'enceinte thermique, représentant un dispositif de mesure de l'émission acoustique.

Figure 5 est une vue de détail représentant un montage adapté à exercer une force constante sur les éléments à analyser.

La machine, selon l'invention, comprend essentiellement une enceinte thermique 2 permettant d'isoler les éléments à contrôler de l'influence extérieure, et de les soumettre à des températures variant sur une plage comprise environ entre - 150°C et + 300°C, en fournissant une bonne homogénéité de la température au niveau des éléments. Cette enceinte thermique comprend une cuve 3 en acier inoxydable, comportant une partie centrale de forme cylindrique, fermée à ses extrémités par des fonds bombés vers l'extérieur. Cette cuve 3 est montée à l'intérieur d'une enveloppe 4, également en acier inoxydable, constituée par exemple par deux demi-coquilles assemblées l'une à l'autre, avec interposition d'un matériau 5 isolant rigide. En pratique, l'axe 6 de l'enceinte thermique est horizontal, et la cuve intérieure 3 repose sur l'élément isolant. L'enveloppe extérieure est pour sa part montée sur un support 7 par l'intermédiaire de pieds 8. Sur l'un des fonds de la cuve sont fixées trois tubulures, qui traversent avec jeu l'élément isolant, ainsi que l'enveloppe extérieure. Il s'agit d'une tubulure 9 pour le passage d'un tube 10 d'amenée de fluide cryogénique tel qu'azote liquide, d'un tube axial 12 permettant le passage d'un arbre 13 d'entraînement d'un ventilateur 14 à partir d'un moteur 15, et d'une tubulure 16 permettant le passage d'un conduit en bout duquel est montée, à l'extérieur de l'enveloppe 4, une soupape 17 assurant la régulation de la pression à l'intérieur de la cuve. A l'autre extrémité de la cuve, débouche, dans le fond de celle-ci, une soupape de sécurité 18.

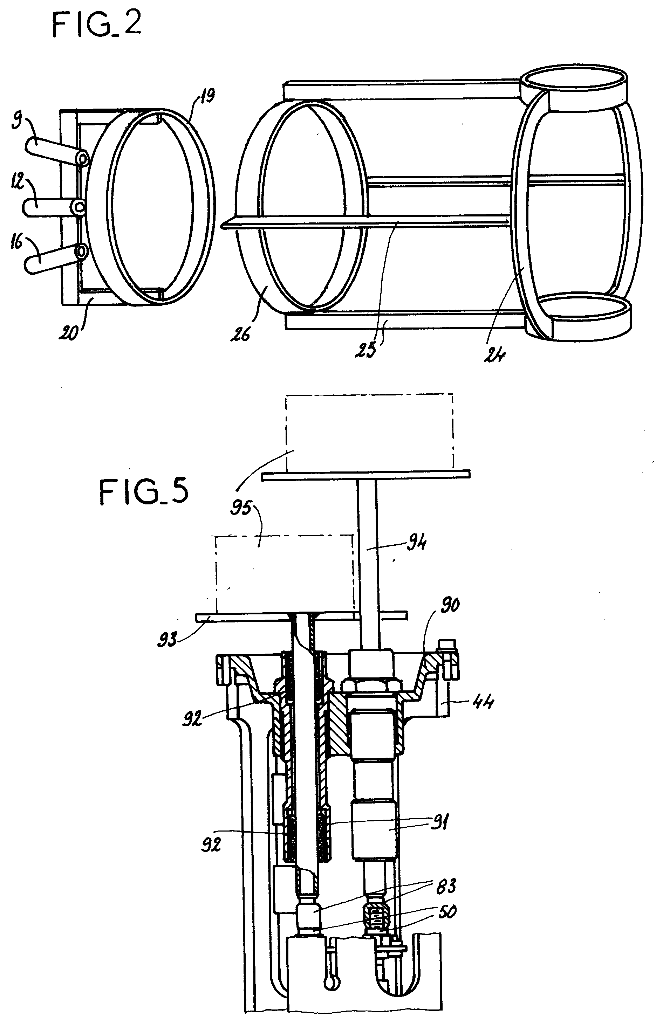

La cuve 3 est équipée, à son extrémité comportant les tubulures 9, 12, 16, d'un anneau 19, périphérique, fixé sur la partie cylindrique à proximité du fond considéré, et portant un étrier 20 sur lequel sont fixées les tubulures 9, 12, 16. En outre, la cuve comporte deux ouvertures diamétralement opposées servant au passage d'un porte-échantillons et de l'instrumentation de mesure. Ces deux ouvertures sont délimitées par des manchettes 22 et 23. Ces manchettes 22 et 23 sont solidaires d'un anneau 24, relié par quatre barres de liaison longitudinales 25, à un anneau 26 qui est monté de façon ajustée et avec possibilité de coulissement axial sur l'anneau 19 portant l'étrier 20 comme montré à la figure 2. Cette structure permet de lier les divers éléments de la machine de façon ferme tout en permettant l'accommodation des dilatations thermiques de la cuve 3, par coulissement des deux anneaux 19 et 26 l'un sur l'autre. Il doit être noté que tout cet ensemble ne possède qu'un point fixe par rapport à l'extérieur constitué par le passage de l'instrumentation, ce qui permet un libre jeu de toutes les autres pièces.

En outre, la cuve comporte une paroi cylindrique 27 en tôle mince, située parallèlement à la paroi cylindrique délimitant la cuve, et à l'intérieur de celle-ci. Cette partie cylindrique 27 s'étend sur la majeure partie de la longueur de la partie cylindrique de la cuve 3. Cette tôle cylindrique 27 délimite avec la cuve, une zone intérieure et centrale 28, et une zone annulaire extérieure 29. Le ventilateur 14 se trouve en regard de la zone centrale 28, cette zone contenant les éléments à contrôler assurant la canalisation du flux de fluide gazeux tel que de l'air depuis le ventilateur, de la gauche vers la droite au dessin, ce flux d'air étant canalisé, à l'extrémité droite de la cuve, à l'aide d'un séparateur 30, vers l'extérieur, et revenant par le volume annulaire 29 dans la partie gauche de la cuve. Une résistance de chauffage 32 est disposée en aval du ventilateur 14 dans le sens de déplacement du flux d'air. Cette ventilation améliore l'échange thermique entre la résistance de chauffage et l'air de l'enceinte, le mélange d'azote et d'air, et notamment l'évaporation de l'azote liquide, ainsi que l'échange thermique entre l'air ambiant de l'enceinte et les éléments à contrôler.

Une unité de contrôle et de commande, non représentée au dessin, assure la régulation de la température, en agissant sur l'amenée d'azote, et sur l'alimentation électrique de la résistance 32. En outre, un calculateur enregistre et traite toutes les informations au cours de la période de contrôle.

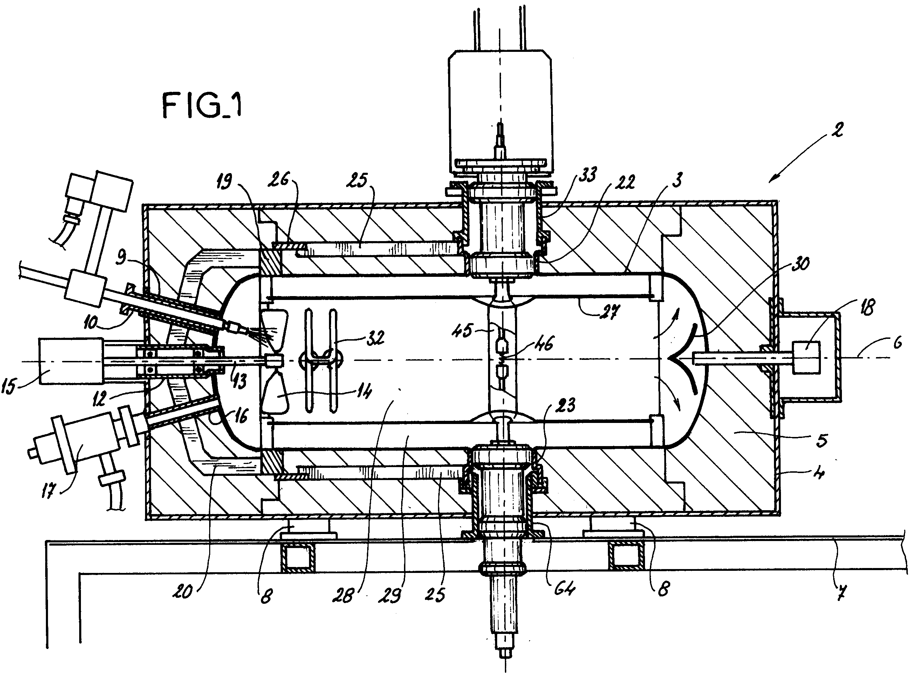

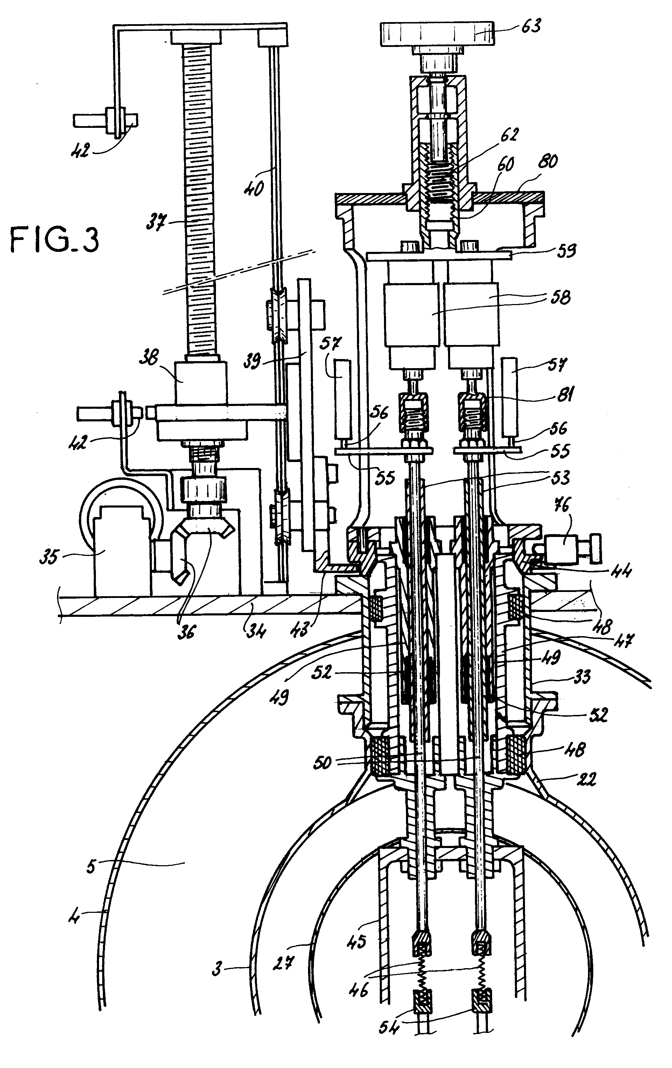

Comme montré aux figures 1 et 3, la manchette 22 délimitant une ouverture débouchant dans la cuve 3, est solidaire d'une partie tubulaire 33 qui la prolonge vers le haut, et qui traverse l'isolant 5, ainsi que l'enveloppe extérieure 4. L'extrémité de cette partie tubulaire 33 porte une plate-forme 34 sur laquelle est monté un dispositif élévateur destiné à déplacer l'instrumentation et les éléments à contrôler lors de leur mise en place dans la cuve et de leur retrait hors de celle-ci. Ce dispositif élévateur comprend un motoréducteur 35 qui entraîne, par l'intermédiaire de deux pignons 36, une vis sans fin 37, sur laquelle est engagé un écrou 38, solidaire d'un chariot 39 guidé sur des rails 40. La vis 37 et les rails 40 sont orientés parallèlement à l'axe de la pièce tubulaire 33. Ce dispositif élévateur est équipé de deux capteurs inductifs 42, destinés à arrêter le moteur 35 en fin de course haute et en fin de course basse.

Comme montré à la figure 3, le chariot 39 est équipé d'une zone 43 de solidarisation au bâti 44 portant l'instrumentation.

Ce bâti 44 est équipé d'un étrier 45 qui, en position de mesure, se trouve à l'intérieur de la cuve 3, et sert au montage des deux éléments 46 à contrôler.

Comme montré à la figure 3, le bâti comporte une partie 47, tubulaire, dont la périphérie est équipée de joints 48, destinés à réaliser l'étanchéité au niveau des éléments 22, 33 traversant les parois de la cuve 3 de l'isolant 5 et de la coque extérieure 4. A l'intérieur de cette partie tubulaire 47, sont montés deux tubes 49, servant chacun au passage d'une tige 50, et susceptibles d'assurer le guidage de chacune de celle-ci à l'aide de deux douilles à billes 52. Le montage de chaque tige 50 dans les douilles à billes 52 est réalisé avec interposition d'une couche 53 d'un matériau isolant thermique.

Les éléments 46 à contrôler, fils ou ressorts, sont en appui ou fixés par leur base contre une butée fixe 54, solidaire du bâti de la machine, et par leur autre extrémité, à une extrémité d'une tige 50. Pour réaliser ces fixations, il convient de ne pas perturber l'échange thermique entre le fluide caloporteur et l'échantillon et, s'il s'agit d'un ressort, d'interdire tout déplacement de celui-ci sans toutefois l'écraser.

L'extrémité libre de chaque tige 50, est située à l'extérieur de l'enceinte thermique. A cette extrémité libre, chaque tige 50 comporte une lame 55 qui en est perpendiculaire, cette lame 55 portant elle-même un doigt 56 en fer doux, constituant l'élément mobile d'un capteur de déplacement magnétique 57. L'extrémité de la tige 50 est également associée, de façon amovible, à un capteur de force 58 de type jauge de contrainte. Les deux capteurs de force 58 des deux tiges 50 sont fixés sur une même platine 59, laquelle est équipée d'un écrou 60, dans lequel est engagée une vis 62 qui, actionnable par un bouton de manoeuvre 63, est montée libre en rotation mais bloquée en translation sur le bâti. Dans une position diamétralement opposée, l'enceinte thermique est équipée d'un dispositif de mesure d'émission acoustique, fixé sur l'étrier 45, et traversant la paroi de la cuve d'une part, mais aussi l'isolant thermique et la paroi extérieure.

A cet effet, sur la manchette 23 délimitant l'ouverture ménagée dans la partie inférieure de la cuve, est fixée une partie tubulaire 64, qui traverse l'isolant rigide 5 ainsi que l'enveloppe extérieure 4, et vient se centrer sur le support 7. La manchette 23 et l'élément tubulaire 64, sont traversés par une partie tubulaire 65 qui, rendue solidaire de l'étrier 45, est montée avec interposition de joints 66 vis-à-vis de la manchette 23 et de la partie tubulaire 64.

Le dispositif de mesure d'émission acoustique, comprend, pour chaque élément, un guide d'ondes 67, qui prend appui, de façon non représentée au dessin, directement contre l'élément 46 à contrôler, sans aucun autre contact et sans toutefois perturber la fixation de celui-ci contre l'élément 54 prévu à cet effet. Ce guide d'ondes s'étend jusqu'à l'extérieur de l'enveloppe extérieure 4, et prend appui contre un capteur d'émission acoustique 68.

Il est prévu des moyens permettant d'assurer la mise en pression de façon réglable du guide d'ondes 67 contre l'élément 46, et des moyens de mise en pression du capteur d'émission acoustique 68 contre le guide d'ondes 67, ces moyens réglant les deux pressions de manière indépendante.

Il ressort de cette structure, que si l'échantillon est placé dans une enceinte susceptible de lui faire subir des variations de température très élevées, par exemple dans une plage de - 150°C à + 300°C, tous les instruments sont situés à l'extérieur de cette enceinte, et ne sont donc pas perturbés par les variations importantes de température.

L'utilisation de cette machine est la suivante. Pour réaliser la mise en place d'éléments à contrôler dans leur support respectif, il est procédé à l'actionnement du moteur 35, pour assurer la remontée de l'instrumentation jusqu'à ce que l'étrier 45 se trouve à l'extérieur de l'enceinte. Avant fixation des éléments à leurs supports respectifs, il a été procédé au soudage du thermo-couple, et des quatre connexions nécessaires à la mesure de la résistivité. L'opérateur fixe alors chaque élément 46 d'une part, par sa base au niveau de la pièce 54 sur le bâti et à son extrémité à une tige 50.

Il est alors procédé à la connexion du thermo-couple à l'appareil de mesure, et au raccordement des quatre fils de connexion destinés à la mesure de résistivité.

Les éléments 46 étant en place, le moteur 35 est actionné dans un sens de déplacement vers le bas du chariot 39, qui correspond à l'introduction de l'étrier 45 à l'intérieur de la cuve. Ce déplacement est réalisé jusqu'en fin de course basse du chariot, position dans laquelle le bâti 44 vient en appui contre le bord supérieur de la pièce tubulaire 33. Au préalable, le positionnement angulaire du porte-éléments vis-à-vis du bâti a été réalisé par engagement d'un doigt 76 à l'intérieur d'un évidement correspondant ménagé dans le bâti.

L'opérateur vérifie que les forces appliquées sur les éléments sont nulles, puis régle les forces de contact entre chaque guide d'ondes 67 et l'élément 46 correspondant, et entre chaque guide d'ondes 67 et le capteur 68 correspondant.

Il est alors possible de procéder aux manipulations souhaitées.

Un type de mesure consiste à imposer une déformation aux éléments 46, et à mesurer la force qu'il faut pour maintenir cette déformation lors d'une variation de température. C'est le montage de type traction/compression qui est utilisé dans ce cas-là. Les éléments sont amenés à la température de démarrage, après quoi la déformation désirée est fixée à l'aide de la vis 62 de mise en charge. Il est ensuite possible de lancer l'exécution d'un cycle de variation de la température.

Un autre type de mesure consiste à imposer une température aux éléments, puis à les soumettre à des essais de traction ou de compression. Dans ce cas, les éléments sont amenés à la température de l'essai en n'étant soumis à aucune contrainte. Ensuite, par actionnement de la vis 63, ils sont déformés progressivement jusqu'à une valeur maximale. Au cours de cette déformation, il est procédé à l'enregistrement de l'émission acoustique de la température, de la résistivité, de la force de déformation et de la déformation. Lorsque la valeur maximale de déformation est atteinte, la charge exercée sur les éléments est réduite jusqu'à obtenir une force imposée nulle. Il est possible de recommencer un autre essai, ou d'arrêter les manipulations ou d'enchaîner plusieurs des manipulations précitées dans un ordre quelconque.

Lorsque l'essai est terminé, l'enceinte thermique est ramenée à la température ambiante, et les éléments sont déchargés par extraction hors de la cuve centrale par l'intermédiaire du dispositif élévateur, et notamment du moteur 35. Il est alors possible de dessouder les fils de mesure de résistivité et de déconnecter le thermo-couple. Les éléments contrôlés sont démontés et d'autres éléments peuvent être installés à leur place.

Si l'on veut appliquer aux éléments une force constante et mesurer la variation de longueur des éléments en fonction d'une variation de température, il convient d'utiliser l'adaptation de montage dont une forme de réalisation est donnée à titre d'exemple à la figure 5. Ce montage s'adapte sur le bâti de l'instrumentation et remplace les pièces 58 à 63 et 80-81. Le démontage de ces pièces se fait en désacouplant les tiges 50 des capteurs 58, en dévissant les écrous 81, et en désolidarisant la pièce 80 du bâti 44 par enlèvement des vis. On peut ensuite placer le montage de force constante en fixant la pièce 90 au bâti 44 en lieu et place de la pièce 80, par vissage, et par accouplement des tiges 50 aux plateaux 93 et 94 à l'aide des écrous 83, identiques aux écrous 81.

Lorsque le montage est en place, les éléments à contrôler n'étant soumis à aucune contrainte sont amenés à la température de début d'essais. On place alors des poids (95) de masses connues sur les plateaux 93 et 94. Ainsi, ces plateaux guidés en translation par les guides à billes 92 appuient sur les éléments par l'intermédiaire des tiges 50, soumettant les éléments à une force de compression constante quelle qu'en soit la température et la déformation des éléments.

Le cycle de température étant programmé, l'opérateur le déclenche. Simultanément, il convient d'actionner le calculateur pour démarrer l'acquisition de l'émission acoustique, de la résistivité, de la température et du déplacement.

Comme il ressort de ce qui précéde l'invention apporte une grande amélioration à la technique existante en fournissant une machine permettant de contrôler la transformation d'un élément, en mesurant plusieurs paramètres, ce contrôle pouvant être effectué dans une plage de température très large, sans que l'instrumentation soit détériorée, avec la plus parfaite fiabilité grâce aux possibilités de dilatation des éléments qui subissent la température.

Comme il va de soi, l'invention ne se limite pas à la seule forme d'exécution de cette machine, décrite ci-dessus à titre d'exemple, elle en embrasse au contraire toutes les variantes.

## Revendications

**1.-** Machine pour le contrôle des caractéristiques de matériaux présentant une transformation de phase, caractérisée en ce qu'elle comprend une enceinte (2) isolée thermiquement, à laquelle sont associés une unité de commande, un générateur de température (10, 32) un dispositif (14) d'homogénéisation de la température, un porte-échantillons (45) disposé à l'intérieur de l'enceinte, et des organes de mesure, des moyens étant prévus pour assurer le montage des différents éléments de commande ou de contrôle fixés sur l'enceinte, tout en permettant les dilatations thermiques de l'enceinte, sous l'effet des différences de température.

**2.-** Machine selon la revendication 1, caractérisée en ce que l'enceinte (2) isolée thermiquement comprend une cuve en acier (3) de forme générale cylindrique comportant des fonds bombés vers l'extérieur, logée à l'intérieur d'une enveloppe métallique (4) avec interposition de matériau isolant rigide (5), l'un des fonds de la cuve étant équipé d'une part, de tubulures (9, 12, 16) traversant avec jeu l'isolant (5) et l'enveloppe (4) et destinées respectivement à l'amenée de fluide cryogénique, au passage d'un arbre d'entraînement (13) d'un ventilateur (14) assurant la circulation de fluide gazeux à l'intérieur de l'enceinte et l'homogénéisation de la température, ces trois tubulures étant fixées également sur une pièce (20) en forme d'étrier solidaire d'un anneau (19) fixé sur la partie cylindrique de la cuve (3), à proximité de ce fond, et d'autre part, d'au moins une ouverture radiale pour le passage d'un porte-échantillons et de l'instrumentation de mesure, chaque ouverture étant délimitée par une manchette (22, 23) les différentes manchettes (22, 23) étant solidaires d'un anneau (24) qui entoure la cuve et qui est relié par des barres longitudinales (25) à un deuxième anneau (26) qui est monté de façon ajustée et avec possibilité de coulissement axial sur l'anneau (19) portant l'étrier.

**3.-** Machine selon la revendication 2, caractérisée en ce que la cuve (3) est d'axe horizontal, son enveloppe (4) repose sur un support (7), et elle comporte deux ouvertures diamétrales pour le passage de l'instrumentation dont une partie est elle-même fixée sur le support.

**4.-** Machine selon l'une quelconque des revendications 2 et 3, caractérisée en ce que l'enceinte thermique comporte une paroi cylindrique (27) en tôle mince qui, s'étendant dans la partie cylindrique de la cuve (3) à l'intérieur de celle-ci, et délimitant une zone centrale (28) et une zone annulaire (29), comporte des ouvertures pour le passage des échantillons et de l'instrumentation, le ventilateur (14) étant disposé en regard de la zone centrale (28), le fond opposé à celui du côté duquel se trouve le ventilateur étant équipé d'un séparateur de flux (30) favorisant le retour de l'air par la zone annulaire (29), une résistance de chauffage (32) étant disposée dans la zone centrale (28), en aval du ventilateur (14), dans le sens d'écoulement du flux d'air.

**5.-** Machine selon l'une quelconque des revendications 2 à 4, caractérisée en ce que la manchette (22) solidaire de la cuve et tournée vers le haut est prolongée par une partie tubulaire (33) qui, traversant l'isolant (5) et l'enveloppe extérieure (4), est équipée, à l'extérieur de cette dernière, d'une plate-forme (34) sur laquelle est monté un dispositif élévateur destiné à déplacer l'instrumentation et les échantillons lors de leur mise en place dans la cuve et de leur retrait hors de celle-ci.

**6.-** Machine selon la revendication 5, caractérisée en ce que le dispositif élévateur comprend un moto-réducteur (35) entraînant une vis sans fin (37) d'axe parallèle à celui de l'instrumentation et sur laquelle est monté un écrou (38) solidaire d'un chariot (39) qui, guidé sur des rails (40) parallèles à la vis, est bloqué en translation sur le bâti (44) portant l'instrumentation, des capteurs inductifs (42) étant prévus pour commander l'arrêt du moteur en fin de course haute et basse du chariot.

**7.-** Machine selon la revendication 6, caractérisée en ce que le bâti portant l'instrumentation est équipé d'un étrier (45) qui, en position de mesure, se trouve à l'intérieur de la cuve (3), et sert au montage de chaque élément (46) à contrôler, par appui ou par fixation de l'extrémité de celui-ci opposée au côté duquel se trouve le dispositif élévateur, l'autre extrémité de chaque élément étant en appui contre ou fixé à l'extrémité d'une tige (50) d'orientation générale radiale par rapport à l'enceinte montée coulissante par rapport au bâti (44) et dont l'autre extrémité, située du côté de l'élévateur, est équipée d'un dispositif permettant son déplacement axial et est associée à un capteur de force (58) et à un capteur de déplacement (57).

**8.-** Machine selon la revendication 7, caractérisée en ce que l'extrémité de la tige (50) située du côté opposé à l'élément (46) à contrôler est fixée par l'intermédiaire d'un capteur de force (58) à un dispositif vis-écrou (60, 62), dont l'un (60) des éléments est associé au support du capteur de force (58) et dont l'autre élément (62) est associé au bâti (44), afin de transformer une rotation imposée en translation.

**9.-** Machine selon la revendication 8, caractérisée en ce que dans le cas où la machine est équipée pour contrôler deux éléments (46) disposés parallèlement l'un à l'autre, les deux capteurs de force (58) qui leur sont associés sont montés sur une platine unique (59) déplaçable en translation de façon contrôlée.

**10.-** Machine selon l'une quelconque des revendications 7 à 9, caractérisée en ce que chaque tige (50) dont une extrémité est associée à un élément (46) formant échantillon est équipée, à proximité de son extrémité située à l'extérieur de l'enceinte, d'une lame (55) qui lui est perpendiculaire et qui porte la partie mobile (56) d'un capteur de déplacement (57).

**11.-** Machine selon la revendication 10, caractérisée en ce que chaque capteur de déplacement (57) est de type magnétique, et réagit au déplacement d'un noyau de fer doux (56), qui est solidaire de la tige (50) dont le déplacement est à mesurer.

**12.-** Machine selon l'une quelconque des revendications 7 à 11, caractérisée en ce que la partie (47) du bâti traversant l'enveloppe extérieure, le matériau isolant et la paroi de la cuve, est tubulaire et montée avec interposition de joints (48) dans les éléments tubulaires fixes (22, 33) traversant ces éléments, cette partie tubulaire (47) comportant elle-même un tube de guidage (49) de chaque tige (50), dans lequel celle-ci est montée avec interposition de douilles à billes (52), un isolant thermique (53) étant prévu entre la tige (50) et les douilles (52).

**13.-** Machine selon l'une quelconque des revendications 7 à 12, caractérisée en ce qu'elle est équipée, du côté de la cuve (3) diamétralement opposé à celui comportant l'instrumentation de mesure des déformations, d'un dispositif de mesure d'émission acoustique, monté sur l'étrier (45) portant le ou les éléments à contrôler, par l'intermédiaire d'un bâti comportant, dans sa zone de traversée de la paroi de la cuve, de l'isolant thermique et de l'enveloppe, une partie tubulaire (65) montée avec interposition de joints (66) dans les éléments tubulaires fixes (23, 64) traversant ces éléments.

**14.-** Machine selon la revendication 13, caractérisée en ce que le capteur (68) d'émission acoustique est monté à l'extérieur de l'enceinte (2) contenant l'élément (46) à contrôler, les ondes ultra-sonores étant guidées de l'élément au capteur par un guide d'ondes (67) en appui sous une force de tension réglable d'une part, sur l'élément (46) et d'autre part, sur le capteur (68), des moyens étant prévus pour réaliser un montage permettant, d'une part, de découpler le guide d'ondes du bâti de la machine pour s'affranchir des vibrations parasites, et d'autre part, de régler indépendamment les forces d'appui du guide d'ondes d'un côté sur l'élément à contrôler et de l'autre sur le capteur d'émission acoustique.

**15.-** Machine selon l'une quelconque des revendications 7 à 14, caractérisée en ce qu'elle est équipée de moyens de montage et de mesure de deux éléments, permettant une mesure différentielle de la résistivité électrique, de l'émission acoustique et de la déformation d'un élément par comparaison avec un élément de référence.

FIG.1

FIG_2

FIG_5

FIG.3

EP 0 480 853 A1

11

# FIG_4

Office européen
des brevets

**RAPPORT DE RECHERCHE EUROPEENNE**

Numero de la demande

EP    91 42 0355

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-4 019 365 (L. WOO)<br>* colonne 1, ligne 5 - ligne 13 *<br>* colonne 3, ligne 27 - ligne 64 *<br>* colonne 4, ligne 49 - colonne 6, ligne 18 *<br>--- | 1,7 | G01N3/18 |
| A | US-A-2 904 993 (F.S. GROVER ET AL.)<br>* colonne 3, ligne 14 - colonne 5, ligne 13;<br>figure 2 *<br>* colonne 9, ligne 9 - ligne 34 *<br>--- | 1,4 | |
| A | GB-A-2 220 270 (CHINA STEEL CORP.)<br>* page 7, ligne 14 - page 10, ligne 26 *<br>--- | 1,7 | |
| A | FR-A-1 434 553 (GESELLSCHAFT FÜR LINDE'S<br>EISMASCHINEN)<br>* page 3, colonne de droite, ligne 68 - page 4,<br>colonne de gauche, ligne 34 *<br>--- | 1 | |
| A | INDUSTRIAL LABORATORY<br>vol. 41, no. 6, Juin 1975, NEW YORK, US<br>pages 941 - 943;<br>G.A. GOGOTSI ET AL.: 'Device for investigations<br>into strength and deformability of refractory<br>materials at high temperatures'<br>* le document en entier *<br>--- | 1 | **DOMAINES TECHNIQUES RECHERCHES (Int. Cl.5)**<br><br>G01N<br>G01M |
| A | FR-A-2 633 050 (COMMISSARIAT A L'ENERGIE<br>ATOMIQUE)<br>* page 6, ligne 2 - ligne 30 *<br><br>----- | 13,14 | |

Le présent rapport a été établi pour toutes les revendications

| Lieu de la recherche | Date d'achèvement de la recherche | Examinateur |
|---|---|---|
| LA HAYE | 15 JANVIER 1992 | BINDON C.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

13